# EUROPEAN PATENT APPLICATION

(11) **EP 3 040 415 A1**
(43) Date of publication of application: **06.07.2016**
(21) Application number: 14839231.9
(22) Date of filing: 26.08.2014
(51) Int. Cl.: C12N 15/09, C12N 1/15, C12N 1/19, C12N 1/21, C12N 5/10, C12N 9/02, C12P 7/64, A61K 31/202, A61P 3/06, A61P 7/00, A61P 7/02, A61P 9/10, A61P 9/12, A61P 29/00, A61P 35/00

(54) **Omega-3 UNSATURATED FATTY ACID ENZYME AND METHOD FOR PRODUCING EICOSAPENTAENOIC ACID**

(30) Priority: 27.08.2013 JP 2013175757
(71) Applicant: Kyoto University, Kyoto-shi, Kyoto 606-8501 (JP); Nisshin Pharma Inc., Tokyo 101-8441 (JP)
(72) Inventor: OGAWA, Jun, Kyoto-shi Kyoto 606-8501 (JP); SAKURADANI, Eiji, Kyoto-shi Kyoto 606-8501 (JP); ANDO, Akinori, Kyoto-shi Kyoto 606-8501 (JP); SHIMIZU, Sakayu, Kyoto-shi Kyoto 606-8501 (JP); HARATA, Masataka, Ueda-shi Nagano 386-0042 (JP); HIRAMOTO, Shigeru, Saitama 356-8511 (JP)
(74) Representative: Blodig, Wolfgang
(86) International application number: PCT/JP2014/072228
(87) International publication number: WO 2015/029966

(57) **Abstract**

Provided is an ω̅3 desaturase having a high enzymatic activity even at normal temperature. A polypeptide consisting of an amino acid sequence having an identity of 80% or more with the amino acid sequence represented by SEQ ID NO: 2 and having ω̅3 desaturation activity, and a gene therefor.

## Description

### [Technical Field]

The present invention relates to a novel polypeptide having ω̅3 desaturase activity and a gene encoding the polypeptide, and use of these for producing eicosapentaenoic acid.

### [Background Art]

A highly-unsaturated fatty acid is a fatty acid having two or more unsaturated bonds and includes ω̅6 unsaturated fatty acids such as linoleic acid (LA, 18: 2n-6), γ-linolenic acid (GLA, 18: 3n-6) and arachidonic acid (ARA, 20: 4n-6); and ω̅3 unsaturated fatty acids such as α-linolenic acid (ALA, 18: 3n-3), eicosapentaenoic acid (EPA, 20: 5n-3) and docosahexaenoic acid (DHA, 22: 6n-3). The highly-unsaturated fatty acids are not only involved in regulating fluidity of membrane as a major constituent of biological membrane but also important as a precursor of a biofunctional component. ARA and EPA serve as precursors of e.g., prostaglandin, thromboxane and leukotriene in higher animals; whereas DHA is a highly-unsaturated fatty acid present most abundantly in the brain. EPA has physiological effects, such as a platelet aggregation inhibitory effect, a hypotensive effect, an anti-arteriosclerotic effect, a blood viscosity-lowering effect, a blood pressure-lowering effect, an anti-inflammatory effect, an anti-tumor effect and used in various fields including pharmaceuticals, foods, cosmetics and animal feeds. Recently, in view of lifestyle-related disease prevention, active intake of ω̅3 unsaturated fatty acids is recommended. Likewise, ω̅3 unsaturated fatty acids are lipid molecular species significantly increased in demand.

DHA and EPA of living bodies are not only taken from food but also biosynthesized from ALA in some organisms. However, since ALA cannot be biosynthesized in humans, DHA and EPA are nutritionally essential fatty acids for humans. EPA is abundantly contained mainly in oils of fish such as cod, herring, mackerel, salmon, sardine and krill; psychrotrophic marine bacteria such as *Shewanella livingstonensis;* and algae such as *Labyrinthulomycetes.* Methods for extracting or purifying EPA from these biological resources have been known. The most common method is purification of EPA from fish oil. However, the EPA content in fish oil is low. In addition to this problem, depending upon the extraction or purification method, fish odor sometimes remains in EPA derived from fish oil, and the content of erucic acid, which is said as a cause of a heart disease, is increased.

Recently, oleaginous microorganisms accumulating lipid within cells, have drawn attention in connection with energy problems and methods for microbiologically producing various types of lipids have been developed. For example, studies on a method for producing a highly-unsaturated fatty acid using microorganisms of a filamentous fungus belonging to the genus *Mortierella* have been conducted. *Mortierella* microorganisms are known to have an ω̅3 or ω̅6 highly unsaturated fatty acid metabolic pathway to produce EPA (Non Patent Literature 1). Patent Literature 1 discloses a method for producing EPA by culturing an EPA-producing *Mortierella* microorganism. Patent Literature 2 discloses a method for producing ARA and EPA using a mutant strain obtained by subjecting *Mortierella alpina* to a mutation treatment. Patent Literature 3 discloses a method for producing a highly-unsaturated fatty acid such as EPA by using a transformed strain obtained by introducing a gene encoding an ω̅3 unsaturated polypeptide isolated from *Mortierella alpina* into a yeast.

However, the ω̅3 desaturase of a *Mortierella* microorganism has a low optimum temperature and does not sufficiently function at normal temperature (about 20°C) where the microorganism easily proliferates. For the reason, even if the *Mortierella* microorganism is cultured at normal culture temperature, EPA cannot be efficiently produced. In addition, since the ω̅3 desaturase of the *Mortierella* microorganism preferentially acts on a fatty acid having 18 carbon atoms, it was difficult to efficiently produce EPA having 20 carbon atoms by the conventional method using the *Mortierella* microorganism.

In the circumstances, it has been desired to develop an ω̅3 desaturase capable of efficiently synthesizing EPA from a fatty acid having 20 carbon atoms (e.g., ARA). An ω̅3 desaturase isolated from *Saprolegnia diclina* is disclosed in Patent Literature 4; Δ17 desaturase isolated from *Phytophthora ramorum* in Patent Literature 5; and Δ17 desaturase isolated from *Pythium aphanidermatum* in Patent Literature 6.

### [Citation List]

### [Patent Literatures]

[Patent Literature 1] JP-A-63-14697
[Patent Literature 2] JP-A-11-243981
[Patent Literature 3] JP-A-2006-055104
[Patent Literature 4] JP-A-2005-515776
[Patent Literature 5] JP-A-2009-534032
[Patent Literature 6] JP-A-2010-508019

### [Non Patent Literature]

[Non Patent Literature 1] Appl. Microbiol. Biotecnol., 1989, 32: 1-4

### [Summary of Invention]

### [Technical Problem]

The present invention relates to providing an ω̅3 desaturase having a high enzymatic activity even at normal temperature of 20°C or more and an oleaginous cell having the ω̅3 desaturase and capable of producing a fat and oil containing EPA in a high concentration effectively. The present invention further relates to providing an industrial production means for an EPA-rich fat and oil using the lipid production cell.

### [Solution to Problem]

The present inventors conducted various investigations. As a result, they found a novel ω̅3 desaturase having a high ω̅3 desaturation activity even at normal temperature and a gene encoding the ω̅3 desaturase. The present inventors further found that productivity of EPA and other ω̅3 unsaturated fatty acids at normal temperature is improved in a transformed cell having the gene encoding the ω̅3 desaturase introduced therein.

More specifically, the present invention provides a polypeptide consisting of an amino acid sequence having an identity of 80% or more with the amino acid sequence represented by SEQ ID NO: 2 and having ω̅3 desaturation activity.

The present invention also provides a polynucleotide encoding the polypeptide.

The present invention also provides a vector comprising the polynucleotide.

The present invention also provides a transformed cell having the polynucleotide introduced therein.

The present invention also provides a method for producing an eicosapentaenoic acid-containing lipid, comprising culturing a cell expressing the polypeptide.

The present invention also provides a method for producing eicosapentaenoic acid, comprising purifying the eicosapentaenoic acid-containing lipid produced by the aforementioned method.

### [Advantageous Effects of Invention]

The ω̅3 desaturase of the present invention has a high ω̅3 desaturation activity at normal temperature of 20°C or more where cells easily proliferate and plays a role in biological synthesis of an ω̅3 unsaturated fatty acid such as EPA. Thus, if a cell expressing the ω̅3 desaturase of the present invention is cultured, an ω̅3 unsaturated fatty acid such as EPA can be efficiently produced within the microorganism. EPA is an important highly-unsaturated fatty acid used in various fields including pharmaceuticals, foods, cosmetics and animal feeds and the present invention applicable to industrial-scale production of EPA is extremely useful in these fields.

### [Brief Description of Drawings]

[Figure 1] Figure 1 shows fatty acid biosynthetic pathway in *M. alpina* 1S-4.
[Figure 2] Figure 2 shows a binary vector for transforming *M. alpina.*

### [Description of Embodiments]

In the specification, unless otherwise specified, "one or more" used in deletion, substitution, addition or insertion of an amino acid(s) or a nucleotide(s) in an amino acid sequence or a nucleotide sequence can refer to, for example, 1 to 20, preferably 1 to 10, more preferably 1 to 5, further preferably 1 to 4, still further preferably 1 to 3 and further more preferably 1 or 2. In the specification, the "addition" of an amino acid(s) or a nucleotide(s) includes addition of one or more amino acids or nucleotides to one end and both ends of a sequence.

In the specification, the identity of amino acid sequences or nucleotide sequences can be determined based on algorithm BLAST (Pro. Natl. Acad. Sci. USA, 1993, 90: 5873-5877) by Karlin and Altschul or FASTA (Methods Enzymol., 1990, 183: 63-98). Based on the algorithm BLAST, programs called as BLASTN and BLASTX have been developed (J. Mol. Biol., 1990, 215: 403-410). When a nucleotide sequence is analyzed by BLASTN based on BLAST, parameters, for example, Score = 100 and wordlength = 12, are used. Furthermore, when an amino acid sequence is analyzed by BLASTX based on BLAST, parameters, for example, score = 50 and wordlength = 3, are used. When BLAST and Gapped BLAST program are used, default parameters of each program are used. Specific manners of these analysis methods are known (see, www.ncbi.nlm.nih.gov).

In the specification, "stringent conditions" refer to the conditions where nucleotide sequences having a high identity, for example, 90% or more, 95% or more, 98% or more, or 99% or more are mutually hybridized; however, nucleotide sequences having a lower identity are not mutually hybridized. More specifically, the "stringent conditions" in the specification refer to conventional washing conditions employed in Southern hybridization, i.e., washing conditions of a temperature and a salt concentration such as 60°C and 1 x SSC, 0.1% SDS, preferably, 0.1 x SSC, 0.1% SDS; and further preferably, 68°C, 0.1 x SSC, 0.1% SDS, and washing times: once and more preferably 2 or 3 times.

In the specification, "corresponding position" or "corresponding region" of a target amino acid sequence or nucleotide sequence to a specific position or region on a specific amino acid sequence or nucleotide sequence can be determined by aligning, the target amino acid sequence or nucleotide sequence with the specific sequence serving as a reference (reference sequence) such that conservative amino acid residues or nucleotides present in individual amino acid sequences or nucleotide sequences have a maximum homology. Alignment can be carried out by use of a known algorithm and its procedure is known to those skilled in the art. For example, alignment, although it can be manually performed based on the Lipman-Pearson method as described above, can be performed by using the Clustal W multiple alignment program (Thompson, J. D. et al., 1994, Nucleic Acids Res., 22: 4673-4680) as default. As "Clustal W", those on the web site of, for example, the European Bioinformatics Institute (: EBI [www.ebi.ac.uk/index.html]) and the web site (DDBJ[www.ddbj.nig.ac.jp/Welcome-j.html]) of the DNA Data Bank of Japan managed by the National Institute of Genetics, can be used.

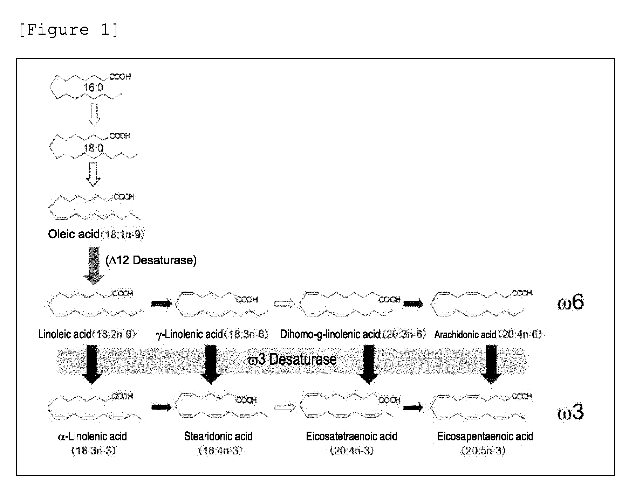

In the specification, the "ω̅6 highly unsaturated fatty acid metabolic pathway" refers to a metabolic pathway for producing an ω̅6 highly-unsaturated fatty acid such as γ-linolenic acid (GLA, 18: 3n-6), dihomo-γ-linolenic acid (DGLA, 20: 3n-6) and arachidonic acid (ARA, 20: 4n-6) from linoleic acid (LA, 18: 2n-6); and the "ω̅3 highly unsaturated fatty acid metabolic pathway" refers to a metabolic pathway for producing an ω̅3 highly-unsaturated fatty acid such as stearidonic acid (SDA, 18: 4n-3), eicosatetraenoic acid (ETA, 20: 4n-3) and eicosapentaenoic acid (EPA, 20: 5n-3) from α-linolenic acid (ALA, 18: 3n-3) (see, Figure 1). In the specification, the "highly-unsaturated fatty acid" refers to a long-chain fatty acid having 18 or more carbon atoms and 2 or more unsaturated bonds.

In the specification, the "desaturation activity" refers to an activity of introducing a carbon-carbon double bond into a fatty acid chain; and the "desaturase" refers to a protein or polypeptide having the desaturation activity. The desaturation activity and desaturase are subdivided based on the position on a fatty acid to which a carbon-carbon double bond is introduced by the activity. For example, the "ω̅3 desaturation activity" refers to an activity of introducing a double bond between the third and fourth carbon from the ω̅ end of a fatty acid and the "ω̅3 desaturase" refers to an enzyme having the ω̅3 desaturation activity and producing an ω̅3 unsaturated fatty acid. Examples of the ω̅3 desaturase may include a conversion enzyme from LA (18: 2n-6) to ALA (18: 3n-3), a conversion enzyme from GLA (18: 3n-6) to SDA (18: 4n-3), a conversion enzyme from DGLA (20: 3n-6) to ETA (20: 4n-3) and a conversion enzyme from ARA (20: 4n-6) to EPA (20: 5n-3).

In the specification, "exhibiting enzymatic activity at normal temperature" means that the optimum temperature of the enzymatic activity is 20°C or more, preferably 20 to 40°C or that the enzymatic activity at 20°C is 70% or more, preferably 80% or more of the enzymatic activity at an optimum temperature.

In the specification, the term "inherent" used in mentioning function, properties and trait of a microorganism is used to express that the function, properties and trait are those present in a wild type of microorganism. In contrast, the term "exogenous" is used to express that the function, properties and trait are not originally present in the microorganism but externally introduced. For example, a gene externally introduced into a microorganism is an exogenous gene. The exogenous gene may be a gene derived from a homogenous microorganism to which the exogenous gene is to be introduced or may be a gene derived from a heterogeneous organism.

The ω̅3 desaturase provided by the present invention is a polypeptide consisting of an amino acid sequence having an identity of 80% or more with the amino acid sequence represented by SEQ ID NO: 2 and having ω̅3 desaturation activity. As an example of the polypeptide, a polypeptide consisting of the following amino acid sequence and having ω̅3 desaturation activity is mentioned.
(A) the amino acid sequence represented by SEQ ID NO: 2;
(B) an amino acid sequence having an identity of 90% or more, preferably 95% or more, more preferably 98% or more, further preferably 99% or more with the amino acid sequence represented by SEQ ID NO: 2;
(C) an amino acid sequence obtained by subjecting the amino acid sequence represented by SEQ ID NO:2 to one or more mutations selected from the group consisting of deletion, substitution, insertion and addition of one or more amino acids;
(D) an amino acid sequence represented by SEQ ID NO: 4;
(E) an amino acid sequence having an identity of 90% or more, preferably 95% or more, more preferably 98% or more, further preferably 99% or more with the amino acid sequence represented by SEQ ID NO: 4, or
(F) an amino acid sequence obtained by subjecting the amino acid sequence represented by SEQ ID NO : 4 to one or more mutations selected from the group consisting of deletion, substitution, insertion and addition of one or more amino acids.

In the amino acid sequence above, the position at which an amino acid(s) is(are) deleted, substituted, inserted and added is not particularly limited as long as the mutated polypeptide maintains ω̅3 desaturation activity.

The ω̅3 desaturase represented by SEQ ID NO: 2 is an enzyme derived from a microorganism of the genus *Pythium* belonging to *Oomycota, Pythiaceae,* namely, *Pythium folliculosum* or *Pythium torulosum.* The ω̅3 desaturase represented by SEQ ID NO: 4 is an enzyme derived from a microorganism of *Pythium sulcatum.* As a result of BLAST analysis, it was found that ω̅3 desaturases represented by SEQ ID NO: 2 and SEQ ID NO: 4 are novel polypeptides having extremely different amino acid sequences from a known protein. The identity of the amino acid sequence between ω̅3 desaturases represented by SEQ ID NO: 2 and SEQ ID NO: 4 is 90.7%. Note that the protein having the most similar amino acid sequence to those of ω̅3 desaturases represented by SEQ ID NO: 2 and SEQ ID NO: 4, is the protein of *Pythium aphanidermatum-derived* Δ17 desaturase (see, Patent Literature 6), which has a sequence identity of 58.5%.

As the ω̅3 desaturase of the present invention, proteins having the aforementioned amino acid sequences (A) to (F), in which individual amino acids may be substituted with amino acids belonging to a group of amino acids analogous in feature, are mentioned. The position and number of amino acids to be substituted with analogous amino acids are not particularly limited as long as the substituted polypeptide maintains ω̅3 desaturation activity. Examples of the amino acids analogous in feature include glycine and alanine; valine, leucine and isoleucine; serine and threonine; aspartic acid and glutamic acid; asparagine and glutamine; lysine and arginine; cysteine and methionine; and phenylalanine and tyrosine.

As a result of gene analysis in *Pythium folliculosum* and *Pythium sulcatum* as mentioned above, the present inventors identified genes encoding the ω̅3 desaturases of the present invention represented by SEQ ID NO: 2 and SEQ ID NO: 4. Each of the genes identified is a novel polynucleotide having two introns in the nucleotide sequence. The present inventors further obtained cDNA molecules of these genes. These cDNA molecules consist of nucleotide sequences represented by SEQ ID NO: 1 and SEQ ID NO: 3 and having no intron sequences. The polynucleotide represented by SEQ ID NO: 1 is derived from *Pythium folliculosum* or *Pythium torulosum* and encodes the ω̅3 desaturase represented by SEQ ID NO: 2. The polynucleotide represented by SEQ ID NO: 3 is derived from *Pythium sulcatum* and encodes ω̅3 desaturase represented by SEQ ID NO: 4.

Accordingly, the present invention further provides a polynucleotide encoding the ω̅3 desaturase of the present invention (hereinafter referred to also as the ω̅3 desaturase gene of the present invention). As an example of the ω̅3 desaturase gene of the present invention, a polynucleotide consisting of the following nucleotide sequence or a complementary strand thereof is mentioned.
(a) a nucleotide sequence represented by SEQ ID NO: 1;
(b) a nucleotide sequence having an identity of 90% or more, preferably 95% or more, more preferably 98% or more, further preferably 99% or more with the nucleotide sequence represented by SEQ ID NO: 1,
(c) a nucleotide sequence obtained by subjecting the nucleotide sequence represented by SEQ ID NO:1 to one or more mutations selected from the group consisting of deletion, substitution, insertion and addition of one or more nucleotides;
(d) a nucleotide sequence hybridizing with the nucleotide sequence represented by SEQ ID NO: 1 under stringent conditions;
(e) a nucleotide sequence represented by SEQ ID NO: 3;
(f) a nucleotide sequence having an identity of 90% or more, preferably 95% or more, more preferably 98% or more, further preferably 99% or more with the nucleotide sequence represented by SEQ ID NO: 3
(g) a nucleotide sequence obtained by subjecting the nucleotide sequence represented by SEQ ID NO: 3 to one more mutations selected from the group consisting of deletion, substitution, insertion and addition of one or more nucleotides; or,
(h) a nucleotide sequence hybridizing with the nucleotide sequence represented by SEQ ID NO: 3 under stringent conditions.

The position in the nucleotide sequence above at which a nucleotide(s) is(are) deleted, substituted, inserted and added is not particularly limited as long as the polypeptide encoded by the mutated polynucleotide maintains ω̅3 desaturation activity.

The ω̅3 desaturase of the present invention can be produced in accordance with a known method, preferably by chemical synthesis or biological synthesis. As an example of the chemical synthesis, a method of extending a peptide chain by sequentially connecting amino acids having a side-chain functional group(s) protected in accordance with an ordinary method may be mentioned. As an example of the biological synthesis, a method including expressing the ω̅3 desaturase of the present invention buy using the ω̅3 desaturase gene of the present invention, isolating the enzyme produced, and, if necessary, further purifying the enzyme, may be mentioned.

Now, the method for biologically synthesizing the ω̅3 desaturase of the present invention will be more specifically described below. First, the ω̅3 desaturase gene of the present invention is prepared. The gene may be produced through chemical synthesis in accordance with a known method or may be isolated from a microorganism such as *Pythium folliculosum* or *Pythium torulosum* as mentioned above. When the ω̅3 desaturase gene of the present invention is isolated from a microorganism, for example, a cDNA library is prepared from total RNA of the microorganism and cDNA of a desired gene of the present invention can be isolated by screening the cDNA library. In screening, a probe or primer is designed based on the nucleotide sequence of the gene of the present invention and cDNA hybridizing with the probe or primer under stringent conditions may be selected. Alternatively, a desired cDNA can be selectively synthesized from the total RNA of the microorganism by a sequence-specific reverse transcription reaction. The cDNA selected can be amplified by a known method such as PCR.

Alternatively, the ω̅3 desaturase gene of the present invention can be prepared by introducing mutation by a known mutagenesis method such as ultraviolet irradiation and site-specific mutagenesis into a gene isolated or synthesized in the aforementioned procedure. For example, mutation is introduced by a known method to a polynucleotide represented by SEQ ID NO: 1 or SEQ ID NO: 3 and isolated from *Pythium folliculosum, Pythium torulosum,* or *Pythium sulcatum* to obtain a mutated polynucleotide. The ω̅3 desaturase gene of the present invention can be obtained by determining the ω̅3 desaturation activity of a polypeptide expressed by the mutated polynucleotide and selecting a gene encoding a polypeptide having a desired activity.

In the ω̅3 desaturase gene of the present invention prepared by the aforementioned procedure, codon optimization is preferably performed in accordance with the frequency of codon usage in a cell into which the gene is introduced and expressed. The information of codons used in various organisms is available in the Codon Usage Database (www.kazusa.or.jp/codon/). For example, if codon optimization of ω̅3 desaturase genes represented by SEQ ID NO: 1 and SEQ ID NO: 3 is performed in accordance with the frequency of codon usage (www.kazusa.or.jp/codon/cgi-bin/showcodon.cgi?species=64518) of *Mortierella alpina,* polynucleotides represented by SEQ ID NO: 5 and SEQ ID NO: 6 are resulted, respectively. Accordingly, as the ω̅3 desaturase gene of the present invention, a polynucleotide obtained by subjecting basically a polynucleotide consisting of any one of the nucleotide sequences described in (a) to (h) to codon optimization in accordance with the frequency of codon usage in each organism, is mentioned.

Next, the ω̅3 desaturase of the present invention is expressed from the ω̅3 desaturase gene of the present invention prepared. Although the enzyme may be expressed in an acellular system, the ω̅3 desaturase gene of the present invention is introduced into a host cell to obtain a transformed cell, and then, the ω̅3 desaturase of the present invention is allowed to express in the transformed cell.

As a host cell to which the ω̅3 desaturase gene of the present invention is to be introduced, microbial cells such as bacterial, fungal and algal cells are preferable but the host cell is not particularly limited to these. The ω̅3 desaturase gene of the present invention can be introduced into a host cell by use of a vector containing the gene. The type of vector to be used for introduction can be appropriately selected depending upon the type of host cell, cloning method, gene expression method and the like. For example, when the ω̅3 desaturase of the present invention is directly expressed from the gene on the vector present outside the genome of a cell, an expression vector is preferably used. The ω̅3 desaturase gene of the present invention is integrated into an appropriate vector. The resultant vector containing the ω̅3 desaturase gene of the present invention is introduced into a host cell. The vector can be introduced into a cell by use of a known method such as an electroporation method, a particle gun (gene gun) method, a competent cell method, a protoplast method, a calcium phosphate coprecipitation method, *Agrobacterium tumefaciens*-mediated transformation (ATMT) method and its modification method (Appl. Environ. Microbiol., 2009,75: 5529-5535). At this time, if an appropriate marker gene is integrated into a vector, a transformed cell having the vector containing the gene of the present invention introduced therein can be screened out based on marker expression as an index.

The ω̅3 desaturase of the present invention is expressed by the transformed cell. The ω̅3 desaturase of the present invention expressed can be isolated or if necessary, purified by a known protein isolation or purification method.

The ω̅3 desaturase of the present invention has a high ω̅3 desaturation activity at normal temperature of, for example, 20°C or more at which cells easily proliferate and can play a role in biological synthesis of an ω̅3 unsaturated fatty acid such as EPA. Thus, when a cell expressing the ω̅3 desaturase of the present invention is cultured at normal temperature, the cell can easily proliferate and an ω̅3 unsaturated fatty acid is biologically synthesized by the ω̅3 desaturase of the present invention expressed in the proliferated cell, with the result that an ω̅3 unsaturated fatty acid such as EPA can be efficiently produced.

Accordingly, the present invention provides a method for producing an EPA-containing lipid, comprising culturing a cell expressing the ω̅3 desaturase of the present invention. The present invention also provides a method for producing EPA, comprising purifying the EPA-containing lipid produced by the EPA-containing lipid production method of the present invention.

The cell for expressing the ω̅3 desaturase of the present invention may be a cell inherently expressing the enzyme or a cell modified so as to express the enzyme. As the cell inherently expressing the ω̅3 desaturase of the present invention, cells of *Pythium folliculosum, Pythium torulosum* and *Pythium sulcatum* are mentioned. As the cell modified so as to express the ω̅3 desaturase of the present invention, the aforementioned transformed cell, which acquirs expressional potency of the ω̅3 desaturase of the present invention by introduction of the ω̅3 desaturase gene of the present invention, is mentioned. The transformed cell may be any cell derived from e.g., plants, bacteria, fungi and algae;and, a microbial cell derived from e.g., bacteria, fungi and algae is preferable. Alternatively, a cell of *Pythium folliculosum, Pythium torulosum* or *Pythium sulcatum* as mentioned above, which is modified so as to improve expression of the ω̅3 desaturase of the present invention, can be used as a method for producing an EPA-containing lipid of the present invention.

In the method for producing an EPA-containing lipid of the present invention, the cell expressing the ω̅3 desaturase of the present invention produces EPA by the action of the ω̅3 desaturase of the present invention. Thus, the cell not only has an expressional potency of the ω̅3 desaturase of the present invention but also inherently has an ability to produce arachidonic acid (ARA) serving as a substrate for the enzyme or is modified so as to produce ARA. Preferably, the cell inherently has an ω̅6 highly unsaturated fatty acid metabolic pathway or is modified so as to have the pathway. More preferably, the cell inherently has an ω̅6 highly unsaturated fatty acid metabolic pathway and an ω̅3 highly unsaturated fatty acid metabolic pathway or modified so as to have the both pathways. As shown in Figure 1, ARA produced via the ω̅6 highly unsaturated fatty acid metabolic pathway is converted into EPA by the action of ω̅3 desaturase. Alternatively, an ω̅6 highly-unsaturated fatty acid such as LA, GLA and DGLA is converted into an ω̅3 highly-unsaturated fatty acid such as ALA, SDA and ETA by the action of an ω̅3 desaturase and then EPA is produced from each of these ω̅3 highly-unsaturated fatty acids via the ω̅3 highly unsaturated fatty acid metabolic pathway.

As a preferable example of the cell to be used in the method for producing an EPA-containing lipid of the present invention, oleaginous microorganisms having a potency of expressing the ω̅3 desaturase of the present invention and an ω̅6 highly unsaturated fatty acid metabolic pathway and to enable production of arachidonic acid are mentioned. More preferably, the oleaginous microorganism further has an ω̅3 highly unsaturated fatty acid metabolic pathway. Examples of such an oleaginous microorganism include *Pythium folliculosum, Pythium torulosum* and *Pythium sulcatum,* as mentioned above; and an oleaginous microorganism having an ability to produce arachidonic acid via the ω̅6 highly unsaturated fatty acid metabolic pathway, preferably an oleaginous microorganism further having an ω̅3 highly unsaturated fatty acid metabolic pathway and modified so as to express the ω̅3 desaturase of the present invention.

The oleaginous microorganism modified so as to express the ω̅3 desaturase of the present invention can be obtained by introducing the ω̅3 desaturase gene of the present invention into an oleaginous microorganism having an ω̅6 highly unsaturated fatty acid metabolic pathway and an ability to produce arachidonic acid, preferably further having an ω̅3 highly unsaturated fatty acid metabolic pathway. Introduction of the ω̅3 desaturase gene of the present invention into the oleaginous microorganism can be carried out in accordance with the aforementioned procedure for introducing a gene to a host cell. Specific procedure thereof will be described below.

Examples of the oleaginous microorganism to which the ω̅3 desaturase gene of the present invention is to be introduced include, but are not limited to, *Pythium* microorganisms, yeasts and filamentous fungi such as *Mortierella, Mucor* and *Umbelopsis,* preferably, *Mortierella* microorganisms such as *Mortierella alpina* (hereinafter sometimes referred to as *M. alpina), Mortierella chlamydospora, Mortierella elongata, Mortierella exigua, Mortierella hygrophila, Mortierella epigama, Mortierella acrotona, Mortierella minutissima, Mortierella lignicola, Mortierella clonocystis, Mortierella* nana, *Mortierella humicola, Mortierella bainieri, Mortierella hyaline, Mortierella globalpina, Umbelopsis nana* and *Umbelopsis isabellina;* and more preferably, *Mortierella alpine (M. alpine), Mortierella·clonocystis, Mortierella nana, Mortierella humicola, Mortierella bainieri, Mortierella hyaline* and *Mortierella globalpina.*

The oleaginous microorganism to which the gene of the present invention is to be introduced may be a mutant strain of microorganism of the *Pythium* such as *Pythium folliculosum, Pythium torulosum* and *Pythium sulcatum,* yeasts and the *Mortierella* as long as it has an ω̅6 highly unsaturated fatty acid metabolic pathway and has an ability to produce arachidonic acid. Since the mutant strain needs not to express an ω̅3 desaturase except the ω̅3 desaturase of the present invention, it may be a strain defective in ω̅3 desaturase which the microorganism inherently has. Examples of such a mutant strain and a defective strain include *M. alpina* 1S-4 (Agric. Biol. Chem., 1987, 51 (3): 785-790) and *M. alpina* ST1358 (Biosci. Biotechnol. Biochem., 2010, 74: 908-917).

The mutant strain and deficient strain of the oleaginous microorganism can be obtained by a conventional method, for example, a treatment with a mutagen such as ethyl methane sulfonate (EMS), methyl methane sulfonate (MMS), N-methyl-N-nitro-N-nitrosoguanidine (J. Gen. Microbiol., 1992, 138: 997-1002), 5-bromodeoxyuridine (BrdU), cisplatin and mitomycin C; and mutation induction by e.g., radiation irradiation, ultraviolet irradiation and high heat treatment; or e.g., suppression of gene expression by RNAi.

The ω̅3 desaturase gene of the present invention may be introduced into the genome of the aforementioned microorganism or integrated into an expression vector and introduced in a cell outside the genome. In either case, the ω̅3 desaturase gene of the present invention is preferably introduced together with the vector having the gene integrated therein. The vector to be used for gene transfer can be appropriately selected by those skilled in the art depending upon the type of microorganism to which the gene is to be introduced, the cloning method and gene expression method and the like. Examples of the vector for use in introducing a gene into the *Mortierella* microorganism outside the genome thereof include pD4 vector (Appl. Environ. Microbiol., 2000, 66 (11): 4655-4661), pDZeo vector (J. Biosci. Bioeng., 2005, 100 (6): 617-622), pDura5 vector (Appl. Microbiol. Biotechnol., 2004, 65 (4): 419-425), pDX vector (Curr. Genet., 2009, 55 (3): 349-356) and pBIG3ura5 (Appl. Environ. Microbiol., 2009, 75: 5529-5535).

It is preferable that the aforementioned vectors contain a promoter sequence or a transcription termination signal sequence for expressing the gene of the present invention integrated or a selective marker gene for selecting a transformant having a desired gene introduced therein. As the promoter, a hyper-expression promoter is preferable. Examples of a preferable hyper-expression promoter for a *Mortierella* microorganism include *M*. *alpina*-derived PP3 promoter and SSA2 promoter, and modified promoters thereof having a substitution, a deletion or an addition to the sequences of these promoters. The hyper-expression promotor is not limited to these as long as the gene introduced can be highly expressed. Examples of the selective marker gene include drug resistant genes such as a kanamycin resistant gene, a streptomycin resistant gene, a carboxin resistant gene, a Zeocin resistant gene and a hygromycin resistant gene; genes compensating an auxotrophic mutation of an amino acid such as leucine, histidine, methionine, arginine, tryptophan and lysine; and genes compensating an auxotrophic mutation of a nucleic acid base such as uracil and adenine. As an example of the preferable selective marker gene, a gene compensating an auxotrophic mutation of uracil is mentioned. For example, an uracil auxotrophic mutant strain of *M. alpina* (Biosci. Biotechnol. Biochem., 2004, 68: 277-285) has been developed. To such an uracil auxotrophy strain, a selective marker gene such as an orotidine-5'-phosphate decarboxylase gene (ura3 gene) or an orotidylic acid pyrophosphorylase gene (ura5 gene) can be used. The procedure for constructing a vector and the types of reagents, e.g., a restriction enzyme or a ligation enzyme, are not particularly limited. Those skilled in the art can construct a vector in accordance with general technical knowledge or by appropriately using commercial products.

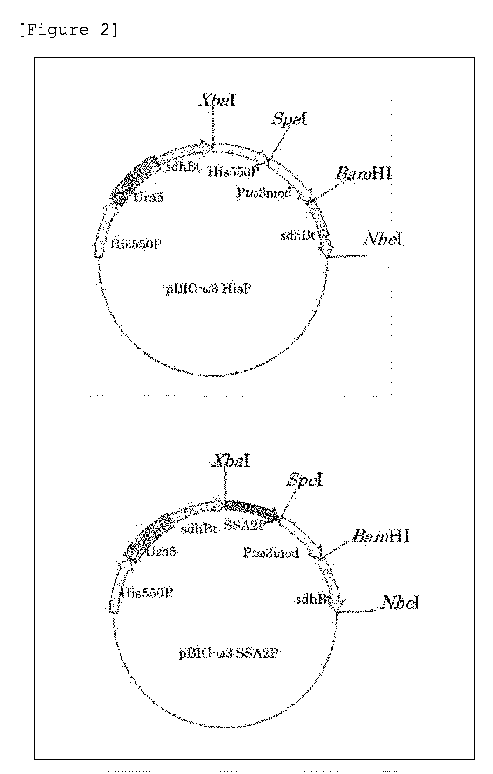

An example of the transformation binary vector, which can be used in introduction of the ω̅3 desaturase gene of the present invention into *M. alpina,* is shown in Figure 2. In the vector, a polynucleotide encoding the ω̅3 desaturase of the present invention (Ptω̅3mod) is ligated downstream of a constitutive hyper-expression promoter, i.e., PP3 promoter or SSA2 promoter, and further, sdhB terminator as a terminator and ura5 gene as a selective marker for a transformant are integrated.

As a method for directly introducing the ω̅3 desaturase gene of the present invention into the genome of a microorganism, homologous recombination is mentioned. The gene of the present invention and a vector having a complementary sequence of a target genome to be introduced are prepared and then the vector is introduced into the microorganism. In this manner, the ω̅3 desaturase gene of the present invention is integrated into the target position on the genome of the microorganism by homologous recombination. In the vector, if necessary, a promoter sequence, a transcription termination signal sequence or a selective marker gene as mentioned above may also be integrated.

A means for introducing a vector into a microorganism may be appropriately selected by those skilled in the art depending upon the type of microorganism and vector. For example, if the vector is introduced into a fungus such as a *Mortierella* microorganism, an electroporation method, a particle gun (gene gun) method, an ATMT method and its modified method (Appl. Environ. Microbiol., 2009, 75: 5529-5535) are mentioned and an ATMT method and its modification method are preferable. As long as a transformant stably maintaining a desired trait can be obtained, the gene transfer method is not limited to these methods.

The cell expressing the ω̅3 desaturase of the present invention to be used in the method for producing an EPA-containing lipid of the present invention may be modified such that the ω̅6 highly unsaturated fatty acid metabolic pathway is activated. For example, if a gene encoding Δ12 desaturase is introduced into the cell to highly express the enzyme, conversion from oleic acid to linoleic acid is accelerated in the cell to activate the ω̅6 highly unsaturated fatty acid metabolic pathway. If the pathway is activated, the amount of ω̅6 highly unsaturated fatty acid serving as a substrate for the enzyme of the present invention is increased, with the result that production of EPA is accelerated.

In the method for producing an EPA-containing lipid of the present invention, the cell expressing the ω̅3 desaturase of the present invention and obtained in the aforementioned procedure is inoculated in a liquid medium or in a solid medium and cultured. The conditions for culture can be optimized by those skilled in the art depending upon the type of cell. For example, if the cell is a fungus, spores or hyphae of the strain or a preculture solution obtained by previously culturing them can be inoculated on the aforementioned culture medium and cultured. Examples of the carbon source for the culture medium include, but are not limited to, glucose, fructose, xylose, saccharose, maltose, soluble starch, corn starch, glycerol, mannitol, a lipids, an alkane and an alkene. Examples of the nitrogen source include, but are not limited to, a natural nitrogen source such as peptone, yeast extract, malt extract, meat extract, casamino acid, corn steep liquor, soybean protein, defatted soybean, cotton seed meal and wheat bran; an organic nitrogen source such as urea; and an inorganic nitrogen source such as sodium nitrate, ammonium nitrate and ammonium sulfate. In addition, a lipid such as soybean oil, coconut oil and corn oil may be added. As the lipid to be added, a fat and oil rich in linoleic acid, such as soybean oil and corn oil, is preferable and soybean oil is more preferable. Moreover, as trace nutrients, mineral salts such as phosphate, magnesium sulfate, iron sulfate and copper sulfate or e.g., vitamin, can be appropriately added. These medium components are not particularly limited as long as they are used in concentrations which do not inhibit growth of the microorganism to be cultured. For example, the concentration of a carbon source in a culture medium is 0.1 to 40 mass%, preferably 1 to 25 mass%; the concentration of a nitrogen source is 0.01 to 10 mass%, preferably 0.1 to 10 mass%. If *M. alpina* or its mutant strain is cultured, e.g., Czapek culture medium (described later), Czapek-dox culture medium, a glucose/yeast extract (hereinafter referred to as "GY") culture medium and SC culture medium can be used. As to the culture medium for *Mortierella* microorganism, a known literature (e.g., International Publication WO No.98/29558) may be used as a reference. The pH of the culture medium may be 4 to 10, preferably 6 to 9. The culture may be carried out in accordance with aerated and agitated culture, shaking culture or static culture.

In order to increase the yield of EPA by accelerating the proliferation of the cell, the cell is preferably cultured at an optimum growth temperature. For example, the cell can be cultured at about 5 to 60°C, preferably about 10 to 50°C, more preferably about 10 to 40°C, further preferably about 20 to 40°C, still further preferably about 20 to 30°C. For example, in the case of *M. alpina* or a mutant strain cell, culture may be performed at about 10 to 40°C, preferably about 20 to 40°C, more preferably about 20 to 30°C. The culture period of the cell may be, for example 2 to 20 days, preferably 2 to 14 days. Note that as to a method for culturing a *Mortierella* microorganism, a known literature (e.g., JP-A6-153970) may be used as a reference.

By culturing a cell expressing the ω̅3 desaturase of the present invention in the above procedure, a lipid containing a large amount of EPA in the cell can be produced. After completion of the culture, the culture solution is treated by a conventional means such as centrifugation and filtration to separate the cells. For example, the culture solution is centrifuged or filtered to remove liquid components. The separated cells are washed and then dried by e.g., lyophilization and airdrying, to obtain dry cells. From the dry cells, a desired lipid can be extracted by a known method such as extraction with an organic solvent. Examples of the organic solvent include a solvent highly dissolving a highly-unsaturated fatty acid and separable from water, such as hexane, ether, ethyl acetate, butyl acetate, chloroform, cyclohexane, benzene, toluene and xylene. These organic solvents can be used in combination. An organic solvent is distilled away from an extract at reduced pressure, etc., to extract a desired lipid. Alternatively, a lipid can be extracted from wet cells without drying cells. The obtained lipid may be further purified by an appropriate common method such as degumming, deacidification, deodorizing, decolorizing, column treatment and distillation.

In the above lipid extract, various types of fatty acids are contained as contaminants other than a target substance, EPA, of the method of the present invention. Thus, the lipid obtained above is further purified to successfully obtain EPA having a further higher purity. EPA can be directly separated from the lipid; and, it is preferable that the fatty acids in the lipid are once converted into ester derivatives with a lower alcohol and then an ester derivative of a desired EPA is separated. Since the ester derivative can be separated by various separation and purification operations depending upon the carbon number, the number and position of double bonds and the like, a desired fatty acid ester derivative can be easily obtained. However, arachidonic acid, which has the same carbon number as EPA but differs by one double bond, is difficult to separate from EPA. For the reason, the content of arachidonic acid in the lipid containing EPA is preferably low. The ester derivative is preferably an ethyl ester derivative. In esterification, a lower alcohol containing an acid catalyst such as hydrochloric acid, sulfuric acid and BF3 or a basic catalyst such as sodium methoxide and potassium hydroxide, can be used. From the obtained ester derivative, an ester derivative of desired EPA can be separated by using column chromatography, low temperature crystallization and urea additional fractionation alone or in combination. The separated EPA ester derivative is hydrolyzed with an alkali, extracted with an organic solvent such as ether and ethyl acetate. In this manner, EPA can be purified. EPA may be purified in the form of a salt.

When the lipid containing a large amount of EPA according to the present invention is produced in an industrial scale, for example, an oleaginous microorganism expressing the ω̅3 desaturase of the present invention is cultured in a large scale in e.g., a tank and filtered by e.g., a filter press to collect cells, and the cells are dried, fractured by e.g., a ball mill and extracted with an organic solvent to obtain the lipid. Other than this, there are many known methods of extracting a component in a microorganism in an industrial scale and purifying EPA from a lipid. These methods can be appropriately modified and used in the method of the present invention.

EPA obtained in the present invention can be used for producing e.g., pharmaceuticals, cosmetics, food and feeds for human or nonhuman animals. Examples of dosage form of the pharmaceutical products include oral agents such as a tablet, a capsule, a granule, a powder, a syrup, a dry syrup, a solution and a suspension; enteral formulations such as an inhalant and a suppository; drops; injections; external preparation; transdermal, transmucosal, and transnasal preparations; inhalation preparations; and patch. Examples of dosage form of the cosmetics include any form of cosmetics usually used, such as cream, emulsion, lotion, suspension, gel, powder, pack, sheet, patch, stick and cake.

The pharmaceutical products or cosmetics contain EPA or a salt thereof as an active ingredient. The pharmaceutical products or cosmetics may further contain a pharmaceutically acceptable carrier or a cosmetically acceptable carrier. Examples thereof include an excipient, a disintegrant, a binder, a lubricant, a surfactant, a pH adjusting agent, a dispersing agent, an emulsifier, a preservative, an antioxidant, a coloring agent, an alcohol, water, a water-soluble polymer, a flavor, a sweetener, a flavoring agent and an acidulant. If necessary, other active ingredients such as medicinal ingredients and cosmetic ingredients may be contained. The pharmaceutical products or cosmetics can be produced by blending a carrier as mentioned above and other active ingredients with EPA or a salt thereof in accordance with the dosage form and preparing in accordance with a conventional method. The content of EPA in the above pharmaceutical products or cosmetics varies depending upon the dosage form and the content usually falls in the range of 0.1 to 99 mass%, preferably 1 to 80 mass%.

The above foods and drinks or feeds contain EPA or a salt thereof as an active ingredient. These foods and drinks or feeds may be health foods, functional foods and drinks, specified health foods and drink, foods and drinks for patients, feeds for e.g., livestock, racehorses and viewing animals and pet foods, which are expected to exert effects such as a platelet aggregation inhibitory effect, a blood triglyceride lowering action, an anti-arteriosclerotic effect, a blood viscosity-lowering effect, a hypotensive effect, an anti-inflammatory effect and anti-tumor effect, as described on labels.

The forms of the above foods and drinks or feeds are not particularly limited and all forms which allow to blend EPA or a salt thereof are included. The foods and drinks may be solid, semi-solid or liquid form, and in various forms such as a tablet, a chewable tablet, a powder, a capsule, a granule, a drink, a gel, a syrup and a liquid diet for tube enteral nutrition. Specific examples of the food and drink forms include tea drinks such as green tea, oolong tea and red tea; beverages such as a coffee drink, a soft drink, a jelly drink, a sports drink, a milk drink, a carbonated drink, a fruit juice drink, a lactic acid bacteria beverage, a fermented milk drink, a powdered beverage, a cocoa beverage, an alcoholic drink and purified water; spreads such as butter, jam, dried food sprinkled over rice and margarine; mayonnaise, shortening, custard cream, dressings, bread, cooked rice, noodles, pasta, miso soup, tofu, cow milk, yogurt, soup or sauces, and confectionery (e.g., biscuits and cookies, chocolate, candy, cake, ice cream, chewing gum, and tablet). Since the feeds can employ almost the same compositions and forms as foods and drinks, the description as to foods and drinks in the specification can be applied to the feeds.

The foods and drinks or feeds as mentioned above can be produced by blending EPA or a salt thereof, other food and drink materials to be used for producing foods and drinks and feeds, nutrients, vitamins, minerals, amino acids, various oils and various additives (e.g., taste components, sweeteners, acidulants such as organic acids, surfactants, pH adjusting agents, stabilizers, antioxidants, dyes, and flavors), and preparing according to conventional methods. Alternatively, the foods or drinks or feeds according to the present invention can be produced by blending EPA or a salt thereof to foods and drinks or feeds usually taken. The content of EPA or a salt thereof in the foods and drinks or feeds varies depending upon the form thereof and the content usually falls in the range of 0.01 to 80 mass%, preferably 0.1 to 50 mass%, more preferably 1 to 30 mass%.

### [Examples]

The present invention will be more specifically described by way of Examples; however, the technical range of the present invention is not limited to the following Examples.

### (Culture medium)

GY culture medium: 2% (w/v) glucose, 1% yeast extract.

Czapek-Dox agar medium: 3% sucrose, 0.2%NaNO₃, 0.1%KH₂PO₄, 0.05%KCl, 0.05%MgSO₄·7H₂O_{,} 0.001%FeSO₄·7H20_{,} 2% agar, pH6.0.

LB-Mg agar medium: 1% tryptone, 0.5% yeast extract, 85 mM NaCl, 0.5 mM MgSO₄·̅7H₂O, 0.5 mM NaOH, 1.5% agar, pH7.0.

Minimal medium (MM) : 10 mM K₂HPO₄, 10 mM KH₂PO₄, 2.5 mM NaCl, 2 mM MgSO₄·7H₂O, 0.7 mM CaCl₂, 9 µM FeSO₄·7H₂O, 4 mM (NH₄) ₂SO₄, 10 mM glucose, pH7.0.

Induction medium (IM): To MM, 0.5% (w/v) glycerol, 200 µM acetosyringone and 40 mM 2-(n-morpholino)ethane sulfonic acid (MES) were added and pH was adjusted to 5.3.

SC culture medium: 5.0 g of Yeast Nitrogen Base w/o Amino Acids and Ammonium Sulfate (Difco), 1.7g of (NH₄)₂SO₄, 20g of glucose, 20 g of agar, 20 mg of adenine, 30 mg of tyrosine, 1.0 mg of methionine, 2.0 mg of arginine, 2.0 mg of histidine, 4.0 mg of lysine, 4.0 mg of tryptophan, 5.0 mg of threonine, 6.0 mg of isoleucine, 6.0 mg of leucine, 6.0 mg of L-phenylalanine.

### Example 1 Identification of ω̅3 desaturase

*Pythium folliculosum* was cultured in GY culture medium (10 mL) at 28°C for 5 days while shaking, and cells were collected. The cells collected were placed in a 2-mL tube and fractured by use of a beads shocker (Yasui Kikai) at 1700 rpm for 10 seconds. This procedure was repeated twice. From fractured cells, mRNA was extracted by use of ISOGEN (Bio-Rad) in accordance with the manufacturer's protocol. The mRNA extracted was subjected to reverse transcription using Prime Script™II High Fidelity RT-PCR Kit (TaKaRa) and primers: [5'-GAAATGGCCGACGTGAACACCTCCTCGC-3' (SEQ ID NO: 7), and 5'-CTATGCGCGCTTGGTGAGCACCTCGC-3' (SEQ ID NO: 8)] to prepare cDNA represented by SEQ ID NO: 1. It was found that the cDNA encodes a polypeptide having the amino acid sequence represented by SEQ ID NO: 2.

mRNA, which was extracted from *Pythium sulcatum,* was subjected to reverse transcription using primers: [5'-CTACGTCAAGGGCAACCTCTCGTCC-3' (SEQ ID NO: 9) and 5'-AAAGCCGAACGACAGCACCATCTTG-3' (SEQ ID NO: 10)], in the same procedure to prepare cDNA represented by SEQ ID NO: 3. It was found that the cDNA encodes a polypeptide having the amino acid sequence represented by SEQ ID NO: 4.

The cDNA (SEQ ID NO: 1) prepared in the above was integrated into a yeast expression vector, pYE22m (Biosci. Biotech. Biochem., 1995, 59: 1221-1228). The vector was introduced into *Saccharomyces cerevisiae* InvSc1 strain (tryptophan auxotrophy oleaginous yeast) by an electroporation method to obtain a transformant. The transformant was cultured in YPD culture medium (polypeptone 20 g, yeast extract 10 g, adenine 0.4 g, agar 20 g and glucose 20 g were diluted in 1,000 mL of water) at 28°C for one day to express the polypeptide from cDNA integrated therein. Note that ω̅3 desaturase inherent in the oleaginous yeast is not expressed in this culture condition. Subsequently, to the culture medium, an ω̅6 unsaturated fatty acid: linoleic acid (LA, 18: 2n-6), γ-linolenic acid (GLA, 18: 3n-6), dihomo-γ-linolenic acid (DGLA, 20: 3n-6), or arachidonic acid (ARA, 20: 4n-6) was added and cultured at 28°C for two days. Thereafter the amounts of the corresponding ω̅3 unsaturated fatty acid: α-linolenic acid (ALA, 18: 3n-3), stearidonic acid (SDA, 18: 4n-3), eicosatetraenoic acid (ETA, 20: 4n-3) or eicosapentaenoic acid (EPA, 20: 5n-3) produced by ω̅3 unsaturation were measured.

As a result, all of LA, GLA, DGLA and ARA were converted into the corresponding ω̅3 unsaturated fatty acids. From this, it was confirmed that polypeptide (SEQ ID NO: 2) encoded by the cDNA (SEQ ID NO: 1) is an enzyme having ω̅3 desaturation activity at normal temperature (Table 1).

**[Table 1]**

| Substrate | Product | Conversion rate (%) ^{*1} |
|---|---|---|
| LA | ALA | 3.03 |
| GLA | SDA | 8.36 |
| DGLA | ETA | 6.90 |
| AA | EPA | 7.86 |

| | | |
|---|---|---|
| *1: Conversion rate % [product amount/(substrate amount + product amount)] x 100 | | |

### Example 2 Preparation of ω̅3 desaturase gene introduction vector

The codon optimization of the nucleotide sequence represented by SEQ ID NO: 1 was performed in accordance with *M. alpina* to obtain the polynucleotide represented by SEQ ID NO: 5. Upstream and downstream of CDS (nucleotide numbers 1 to 1,110) of the polynucleotide represented by SEQ ID NO: 5, SpeI and BamHI sites were constructed and the resultant construct was cloned into a SpMA-RQ (ampR) plasmid. The plasmid prepared was treated with SpeI and BamHI restriction enzymes. The resultant fragment of the gene was ligated to pBIG35 plasmid (pBIG2RHPH2 plasmid provided from Kyoto Prefectural University was modified, described in Appl. Environ. Microbiol., 2009, 75: 5529-5535) containing a constitutive hyper expression promoter, His550 promoter or SSA2 promoter, to construct an expression cassette. The expression cassette was further tandemly ligated to an uracil auxotrophy marker gene (ura 5) to construct a binary transformation vector, pBIG-ω̅3HisP and pBIG-ω̅3SSA2P (Figure 2).

### Example 3 Preparation of ω̅3 desaturase gene-introduced strain

*M. alpina* (uracil auxotrophy strain) was cultured in a 0.05 mg/mL uracil-containing Czapek-Dox agar medium. A culture was recovered and then filtered by Miracloth (Calbiochem) to prepare a spore suspension of *M. alpina.* To the *M. alpina* (uracil auxotrophy strain), the pBIG-ω̅3His vector constructed in Example 2 was introduced in accordance with the ATMT method (Appl.Environ.Microbiol.,2009,75: 5529-5535) described below to prepare an ω̅3 desaturase introduced strain.

The above binary vector pBIG-ω̅3His was introduced by electroporation into Agrobacterium cells (*Agrobacterium tumefaciens* C58C1, provided by Kyoto Prefectural University) and the Agrobacterium cells were cultured in LB-Mg agar medium at 28°C for 48 hours. The vector-containing Agrobacterium cells were screened by PCR method. The vector-containing Agrobacterium cells were cultured in minimum culture medium (MM) for 2 days and centrifuged at 5,800 x g. A fresh induction medium (IM) was added to prepare a suspension. The suspension was subjected to induction culture by using a rotary shaker for 8 to 12 hours, at 28°C until OD₆₆₀ reached 3.7 from 0.4. After completion of the culture, the bacterial suspension (100 µL) was mixed with the same amount of *M. alpina* suspension (10⁸ mL⁻¹) obtained above and applied to a co-culturing medium (the same composition as in IM except that 5 mM glucose was contained in place of 10 mM glucose, and 1.5% agar was contained) on which nitrocellulose membrane (diameter 70 mm; hardened low-ash grade 50, Whatman) was placed, and cultured at 23°C for 2 to 5 days. After completion of the co-culturing, the membrane was transferred to uracil-free SC culture medium containing 50 g/mL cefotaxime, 50g/mL spectinomycin and 0.03% Nile blue A (Sigma), and cultured at 28°C for 5 days. Hyphae from visible fungus colonies were transferred to fresh uracil-free SC culture medium. The cell, which can grow in the uracil-free SC culture medium and cannot grow in GY culture medium containing 5-fluoroorotic acid (5-FOA), was determined as ω̅3 desaturase gene-introduced strain stably maintaining the trait. To select a transformant stably maintaining the trait, the operation was repeated three times.

### Example 4 Preparation of ω̅3 desaturase gene-introduced strain

An ω̅3 desaturase introduced strain was prepared in the same procedure as in Example 3 except that pBIG-ω̅3SSA2P was used as the gene transfer vector.

### Example 5 Production of ω̅3 unsaturated fatty acid by ω̅3 desaturase gene-introduced strain

The ω̅3 desaturase gene introduced *M. alpina* strains obtained in Examples 3 and 4 were each aerobically cultured in a 10 mL of GY culture medium at 28°C for 7 days at 300 rpm. As a control, *M. alpina* strain not having the ω̅3 desaturase gene introduced therein was cultured in the same manner. From each of the culture solutions, cells were collected by suction filtration and dried at 120°C for 3 hours. To the dried cells, a dichloromethane solution (1 mL) containing a 0.5 mg/mL internal standard (saturated fatty acid having 23 carbon atoms which cannot be biosynthesized by *M. alpina)* and a hydrogen chloride methanol (2 mL) were added to methyl-esterify the fatty acid in a warm bath at 55°C for 2 hours. To the reaction solution, distilled water (1 mL) and hexane (4 mL) were added and a hexane layer was extracted. The extract was centrifuged under reduced pressure to recover a fatty acid methyl ester.

The collected sample was dissolved in chloroform and subjected to gas-liquid chromatography (GLC) to determine the fatty acid composition in the sample. GLC was performed by use of GC-2010 (manufactured by Shimadzu Corporation) and capillary column TC70 (0.25 mm x 60 m), manufactured by GL Sciences in the conditions: column temperature: 180°C, vaporizing-chamber temperature: 250°C, detector temperature: 250°C, carrier gas: He, make-up gas: N₂, a H₂ flow rate: 40 mL/min, air flow-rate: 400 mL/min, split ratio: 50, and analysis time: 30 min. The amount of each fatty acid extracted was determined from a peak area value in the GLC chart based on the amount of fatty acid of the internal standard. In this way, the ratio of each fatty acid to total fatty acid amount was obtained.

As a result, in ω̅3 desaturase gene-introduced strains of Example 3 and Example 4, EPA accumulation were 9.1% and 18.1%, respectively. In contrast, in the control strain, EPA was not produced (accumulation could not be determined).

## Claims

1. A polypeptide consisting of an amino acid sequence having an identity of 80% or more with an amino acid sequence represented by SEQ ID NO: 2 and having ω̅3 desaturation activity.

2. The polypeptide according to Claim 1, wherein the amino acid sequence having an identity of 80% or more with the amino acid sequence represented by SEQ ID NO: 2 is the following amino acid sequence:
(A) the amino acid sequence represented by SEQ ID NO: 2;
(B) an amino acid sequence having an identity of 90% or more with the amino acid sequence represented by SEQ ID NO: 2;
(C) an amino acid sequence obtained by subjecting the amino acid sequence represented by SEQ ID NO: 2 to one or more mutations selected from the group consisting of deletion, substitution, insertion and addition of one or more amino acids;
(D) an amino acid sequence represented by SEQ ID NO: 4;
(E) an amino acid sequence having an identity of 90% or more with the amino acid sequence represented by SEQ ID NO: 4, or
(F) an amino acid sequence obtained by subjecting the amino acid sequence represented by SEQ ID NO : 4 to one or more mutations selected from the group consisting of deletion, substitution, insertion and addition of one or more amino acids.

3. A polynucleotide encoding the polypeptide according to Claim 1 or 2.

4. The polynucleotide according to Claim 3 consisting of the following nucleotide sequence:
(a) a nucleotide sequence represented by SEQ ID NO: 1;
(b) a nucleotide sequence having an identity of 90% or more with the nucleotide sequence represented by SEQ ID NO: 1,
(c) a nucleotide sequence obtained by subjecting the nucleotide sequence represented by SEQ ID NO: 1 to one or more mutations selected from the group consisting of deletion, substitution, insertion and addition of one or more nucleotides;
(d) a nucleotide sequence hybridizing with the nucleotide sequence represented by SEQ ID NO: 1 under stringent conditions;
(e) a nucleotide sequence represented by SEQ ID NO: 3;
(f) a nucleotide sequence having an identity of 90% or more with the nucleotide sequence represented by SEQ ID NO: 3
(g) a nucleotide sequence obtained by subjecting the nucleotide sequence represented by SEQ ID NO: 3 to one or more mutations selected from the group consisting of deletion, substitution, insertion and addition of one or more nucleotide;
(h) a nucleotide sequence hybridizing with the nucleotide sequence represented by SEQ ID NO: 3 under stringent conditions; or
(i) a nucleotide sequence obtained by optimizing a codon of each of the nucleotide sequences represented by (a) to (h).

5. A vector comprising the polynucleotide according to Claim 3 or 4.

6. A transformed cell having the polynucleotide according to Claim 3 or 4 introduced therein.

7. A method for producing an eicosapentaenoic acid-containing lipid, comprising culturing a cell expressing the polypeptide according to Claim 1 or 2.

8. A method for producing eicosapentaenoic acid, comprising purifying an eicosapentaenoic acid-containing lipid produced by the method according to Claim 7.


SEQUENCE LISTING

<110> Kyoto University
Nisshin Pharma Inc.

<120> Omega-3 desaturase and method of producing eicosapentaenoic acid

<130> NS0064

<150> JP2013-175757
<151> 2013-08-27

<160> 10

<170> PatentIn version 3.5

<210> 1
<211> 1110
<212> DNA
<213> Pythium folliculosum

<220>
<223> Omega-3 desaturase gene

<400> 1
atggccgacg tgaacacctc ctcgcccgcg gcggcgcccg tggccacccc cgagcccttc 60

gagttcccgt cgctcacgga gctcaagcgc tcgatcccgg cgcgctactt cgagtcgtcg 120

gcgccgcgct cgctcttgtt cacggcgcgc gcgttcgcgt tcgcctacgc gttcggcgcc 180

gcgctcttcc acgcgcgcca gctcccgctc gtggccgagc acgcggcgct ggacgcgctg 240

ctctgcgccg cctacacctt cctccagggc gtcaacatgt ggggcttctt cacgatcggc 300

cacgactgcg gccacggctc gttctcgcgg taccacctgc tcaacttcgt ggtgggcacg 360

ctcactcact cgctcatcct cacgccgtac gaggcctgga agctctcgca ccgcctccac 420

cacaagaaca cgggcaacat cgaccaggac gagatcttct acccgcagcg caaggagact 480

gtgaacgacc cggcccgcaa gatggtgctg tccttcggcc tcgcctggtg gatctacgag 540

atcatcggct tcccgccgcg caccgaggag cacacggagc tccgtgactc gctcttctcg 600

cgccacctca tcacgggctc gctctcggtg ctcgcgcacc tggcctgcgt catgctggcg 660

ttcaacctca cgtacgtgct gggctgggcc accatgggcg tctactactg ggcgccgctc 720

ttcgtcttcg cgtccatgct cgtcatcacg accttcctgc accacaacga cgaggagacg 780

ccgtggtacg ccgactcgga gtggacgtac gtcaagggca acctctcgtc cgtggaccgc 840

gactatggct gggtcgtgaa ccacctgagc cacaacatcg gcacgcacca ggtgcaccac 900

ttgttcccga tcatcccgca ctacaacctc aacggcgcga ccgagtactt ccgcaaggcc 960

ttcccgcacc tcgtgcgcaa gagcgacgac cgcatcctcc cggccttcgt gcgcgtcggc 1020

ctgctctacg tcaagtacgg cgtgatcgag aacgacgcca agcacttttc gctcaaggac 1080

aaccgcgagg tgctcaccaa gcgcgcatag 1110

<210>  2
<211>  369
<212>  PRT
<213>  Pythium folliculosum

<220>
<223>  Omega-3 desaturase

<400>  2

Met Ala Asp Val Asn Thr Ser Ser Pro Ala Ala Ala Pro Val Ala Thr
1               5                   10                  15

Pro Glu Pro Phe Glu Phe Pro Ser Leu Thr Glu Leu Lys Arg Ser Ile
            20                  25                  30

Pro Ala Arg Tyr Phe Glu Ser Ser Ala Pro Arg Ser Leu Leu Phe Thr
        35                  40                  45

Ala Arg Ala Phe Ala Phe Ala Tyr Ala Phe Gly Ala Ala Leu Phe His
    50                  55                  60

Ala Arg Gln Leu Pro Leu Val Ala Glu His Ala Ala Leu Asp Ala Leu
65                  70                  75                  80

Leu Cys Ala Ala Tyr Thr Phe Leu Gln Gly Val Asn Met Trp Gly Phe
                85                  90                  95

Phe Thr Ile Gly His Asp Cys Gly His Gly Ser Phe Ser Arg Tyr His
            100                 105                 110

Leu Leu Asn Phe Val Val Gly Thr Leu Thr His Ser Leu Ile Leu Thr
        115                 120                 125

Pro Tyr Glu Ala Trp Lys Leu Ser His Arg Leu His His Lys Asn Thr
    130                 135                 140

Gly Asn Ile Asp Gln Asp Glu Ile Phe Tyr Pro Gln Arg Lys Glu Thr
145                 150                 155                 160

Val Asn Asp Pro Ala Arg Lys Met Val Leu Ser Phe Gly Leu Ala Trp
                165                 170                 175

Trp Ile Tyr Glu Ile Ile Gly Phe Pro Pro Arg Thr Glu Glu His Thr
            180                 185                 190

Glu Leu Arg Asp Ser Leu Phe Ser Arg His Leu Ile Thr Gly Ser Leu
        195                 200                 205

Ser Val Leu Ala His Leu Ala Cys Val Met Leu Ala Phe Asn Leu Thr
    210                 215                 220

Tyr Val Leu Gly Trp Ala Thr Met Gly Val Tyr Tyr Trp Ala Pro Leu
225                 230                 235                 240

Phe Val Phe Ala Ser Met Leu Val Ile Thr Thr Phe Leu His His Asn
                245                 250                 255

Asp Glu Glu Thr Pro Trp Tyr Ala Asp Ser Glu Trp Thr Tyr Val Lys
            260                 265                 270

Gly Asn Leu Ser Ser Val Asp Arg Asp Tyr Gly Trp Val Val Asn His
        275                 280                 285

Leu Ser His Asn Ile Gly Thr His Gln Val His His Leu Phe Pro Ile
    290                 295                 300

Ile Pro His Tyr Asn Leu Asn Gly Ala Thr Glu Tyr Phe Arg Lys Ala
305                 310                 315                 320

Phe Pro His Leu Val Arg Lys Ser Asp Asp Arg Ile Leu Pro Ala Phe
                325                 330                 335

Val Arg Val Gly Leu Leu Tyr Val Lys Tyr Gly Val Ile Glu Asn Asp
            340                 345                 350

Ala Lys His Phe Ser Leu Lys Asp Asn Arg Glu Val Leu Thr Lys Arg
        355                 360                 365

Ala

<210>  3
<211>  1098
<212>  DNA
<213>  Pythium sulcatum

<220>
<223>  Omega-3 desaturase gene

<400>  3
atggctgcga tcgcctatag ctcgctgccg acggacgcca agccgttcga gttcccgacg      60

ctcacggagc tcaagcggtc gatcccggcg cgctacttcg agtcatcggc gccgcgctcg     120

ctcttcttca cggcgcgcgc gttcgcgttc gcctacgcgt ttggcttcgc gctctggcgc     180

gcgcggcagc tgccggtcgt cgaggcccac gccgcgctcg acgcgctgct ctgcggcacc     240

tacgtcttcc tccagggcgt caacatgtgg ggcttcttca cgatcggcca cgactgcggc     300

cacgggtcgt tctcgcggtc ccacgtgctc aactttgtcg tcggcacgct cacgcactcg 360

ctgatcctca cgccgtacga ggcctggaag ctctcgcacc ggctccacca caaaaacacg 420

ggcaacatcg accaggacga gatcttttac ccgcagcgca aagagacggt caacgacccg 480

gcgcgcaaga tggtgctgtc gttcggcttt gcctggttca tctacgagat catcggcttc 540

ccgccgcgca ccgacgagca caccgagctg cgcgactcgc tgttcgctcg ccacctcctc 600

gcgtgctcgc tctcggtgat cgcccacctc gcgtgcgtca tgctggcgtt caacctcacc 660

tacgtgctgg gctgggccac catgggcgtg tactactggg cgccgctctt cgtgttcgcg 720

tccatgctcg tcatcacgac cttcctgcac cacaacgacg aagagacccc gtggtacgcc 780

gactcggagt ggacctacgt caagggcaac ctctcgtccg tggatcgcga ctacggctgg 840

ctggtcaacc acctgagcca caacattggc acgcaccagg tgcaccactt gttcccgatc 900

atcccgcact acaacctcaa cggcgcgacc gagtacttcc gcaaggcttt cccgcacctc 960

gtccgcaaga gcgacgaccg gatcatcccc gcctttatcc gcgtcggact gctctacgtc 1020

aagtacggcg tgatcgagaa cgacgccaag atgttctcgc tcaaggacaa ccgcgagacg 1080

ctcaccaaga gctcgtag 1098

<210> 4
<211> 365
<212> PRT
<213> Pythium sulcatum

<220>
<223> Omega-3 desaturase

<400> 4

Met Ala Ala Ile Ala Tyr Ser Ser Leu Pro Thr Asp Ala Lys Pro Phe
1               5                   10                  15

Glu Phe Pro Thr Leu Thr Glu Leu Lys Arg Ser Ile Pro Ala Arg Tyr
            20                  25                  30

Phe Glu Ser Ser Ala Pro Arg Ser Leu Phe Phe Thr Ala Arg Ala Phe
        35                  40                  45

Ala Phe Ala Tyr Ala Phe Gly Phe Ala Leu Trp Arg Ala Arg Gln Leu
    50                  55                  60

Pro Val Val Glu Ala His Ala Ala Leu Asp Ala Leu Leu Cys Gly Thr
65                  70                  75                  80

Tyr Val Phe Leu Gln Gly Val Asn Met Trp Gly Phe Phe Thr Ile Gly
                85                  90                  95

His Asp Cys Gly His Gly Ser Phe Ser Arg Ser His Val Leu Asn Phe
            100                 105                 110

Val Val Gly Thr Leu Thr His Ser Leu Ile Leu Thr Pro Tyr Glu Ala
        115                 120                 125

Trp Lys Leu Ser His Arg Leu His His Lys Asn Thr Gly Asn Ile Asp
    130                 135                 140

Gln Asp Glu Ile Phe Tyr Pro Gln Arg Lys Glu Thr Val Asn Asp Pro
145                 150                 155                 160

Ala Arg Lys Met Val Leu Ser Phe Gly Phe Ala Trp Phe Ile Tyr Glu
                165                 170                 175

Ile Ile Gly Phe Pro Pro Arg Thr Asp Glu His Thr Glu Leu Arg Asp
            180                 185                 190

Ser Leu Phe Ala Arg His Leu Leu Ala Cys Ser Leu Ser Val Ile Ala
        195                 200                 205

His Leu Ala Cys Val Met Leu Ala Phe Asn Leu Thr Tyr Val Leu Gly
    210                 215                 220

Trp Ala Thr Met Gly Val Tyr Tyr Trp Ala Pro Leu Phe Val Phe Ala
225                 230                 235                 240

Ser Met Leu Val Ile Thr Thr Phe Leu His His Asn Asp Glu Glu Thr
                245                 250                 255

Pro Trp Tyr Ala Asp Ser Glu Trp Thr Tyr Val Lys Gly Asn Leu Ser
            260                 265                 270

Ser Val Asp Arg Asp Tyr Gly Trp Leu Val Asn His Leu Ser His Asn
        275                 280                 285

Ile Gly Thr His Gln Val His His Leu Phe Pro Ile Ile Pro His Tyr
    290                 295                 300

Asn Leu Asn Gly Ala Thr Glu Tyr Phe Arg Lys Ala Phe Pro His Leu
305                 310                 315                 320

Val Arg Lys Ser Asp Asp Arg Ile Ile Pro Ala Phe Ile Arg Val Gly
                325                 330                 335

Leu Leu Tyr Val Lys Tyr Gly Val Ile Glu Asn Asp Ala Lys Met Phe
            340                 345                 350

Ser Leu Lys Asp Asn Arg Glu Thr Leu Thr Lys Ser Ser
        355                 360                 365

<210>  5
<211>  1110
<212>  DNA
<213>  Pythium folliculosum

<220>
<223>  Omega-3 desaturase gene optimized for codon usage in Mortierella
alpina

<400>  5
atggctgacg tgaacacttc gtcgcctgcc gctgcccctg tcgccacccc cgagcccttc      60

gagttcccct cgttgaccga gctcaagcgc tcgatccctg cccgctactt tgagtcgtcc     120

gcccctcgct ccctcttgtt taccgcccgt gccttcgctt tcgcctacgc ttttggagcc     180

gctctgtttc acgcccgtca gctccccttg gtcgccgagc acgctgctct cgacgctctc     240

ttgtgcgccg cttacacctt cctccaggga gttaacatgt ggggcttctt caccatcggt     300

cacgactgcg gccacggctc cttttcccgt taccacctgt tgaacttcgt ggtcggcacc     360

ctcacccact cgctcattct cactccctac gaggcctgga agctctcgca tcgcctccac     420

cataagaaca ccggcaacat cgaccaggac gagattttct acccccagcg caaggagact     480

gtgaacgacc ctgctcgcaa gatggtcctc tcgtttggcc tcgcctggtg gatctacgag     540

atcatcggct tccctccccg caccgaggag cacactgagc tccgtgattc gctcttctcg     600

cgccatctca tcaccggttc gttgtccgtc ctcgcccacc tggcctgcgt catgttggcc     660

tttaacctca cttacgtgct gggctgggcc accatgggag tctactactg ggctcccttg     720

ttcgtttttg cctccatgct ggttatcacc accttcttgc atcacaacga tgaggagacc     780

ccctggtacg ccgactcgga gtggacctac gtcaagggca acctctcctc cgtcgaccgc     840

gattacggct gggtcgtcaa ccatctgtcc cacaacattg gcactcacca ggtgcatcat     900

ctcttcccta tcatccccca ttacaacttg aacggcgcca ccgagtactt ccgcaaggct     960

ttcccccacc tcgtgcgtaa gtcggatgat cgcatcctcc ctgctttcgt tcgtgttgga    1020

ttgctctacg tgaagtacgg cgtcatcgag aacgacgcca agcattttcc cctgaaggac    1080

aaccgcgagg tcctcaccaa gcgcgcctaa                                     1110

<210>  6
<211>  1098
<212>  DNA
<213>  Pythium sulcatum

<220>
<223>  Omega-3 desaturase gene optimized for codon usage in Mortierella
alpina

<400>  6
atggccgcca tcgcctactc ctccctccct actgatgcta agcccttcga gtttcccact      60

ctgactgagc tgaagcgctc gatccccgct cgttactttg agtcctccgc ccctcgctcc     120

ctcttcttca ccgctcgcgc cttcgctttc gcttacgcct ttggcttcgc cctctggcgt     180

gcccgccagc tccccgtcgt cgaggcccac gctgccttgg acgctctcct ctgcggtacc     240

tacgttttcc tccagggcgt caacatgtgg ggcttcttta ccatcggcca tgactgcggc     300

cacggctcct tctcccgctc ccatgtcctg aacttcgtgg ttggcacttt gactcactcg     360

ctgattctca ccccctacga ggcctggaag ctctcgcacc gcctccatca caagaacacc     420

ggcaacatcg accaggacga gattttctac ccccagcgca aggagactgt gaacgaccct     480

gcccgcaaga tggtgctctc gttcggcttt gcctggttca tctacgagat catcggcttc     540

ccccctcgta ctgacgagca caccgagctg cgcgactcgt tgttcgcccg tcacttgttg     600

gcctgctcgc tctcggtcat cgcccatctc gcctgcgtca tgctggcctt taacctcact     660

tacgtcctcg gctgggccac tatgggcgtc tactactggg ctcctctctt tgtcttcgcc     720

tcgatgctgg ttatcaccac ttttctgcac cacaacgacg aggagacccc ctggtacgcc     780

gactccgagt ggacctacgt gaagggaaac ctctcgtcgg tggaccgcga ttacggctgg     840

ctcgtgaacc acctgtcgca caacattggc acccaccagg tgcatcacct cttccccatc     900

atcccccact acaacctcaa cggcgccact gagtacttcc gcaaggcttt tccccacctc     960

gtgcgcaagt cggacgaccg catcattcct gcctttattc gcgttggact cttgtacgtt    1020

aagtacggcg tcattgagaa cgatgccaag atgttctccc tgaaggataa ccgtgagacc    1080

ttgaccaagt cctcgtaa                                                  1098

<210>  7
<211>  28
<212>  DNA
<213>  Artificial sequence

<220>
<223>  Oligonucleotide as PCR primer

<400>  7
gaaatggccg acgtgaacac ctcctcgc                                         28

<210>  8
<211>  26
<212>  DNA
<213>  Artificial sequence

<220>
<223>  Oligonucleotide as PCR primer

<400>  8
ctatgcgcgc ttggtgagca cctcgc                                           26

<210>  9
<211>  25
<212>  DNA
<213>  Artificial sequence

<220>
<223>  Oligonucleotide as PCR primer

<400>  9
ctacgtcaag ggcaacctct cgtcc                                                25


<210>  10
<211>  25
<212>  DNA
<213>  Artificial sequence

<220>
<223>  Oligonucleotide as PCR primer

<400>  10
aaagccgaac gacagcacca tcttg                                                25